# EUROPEAN PATENT APPLICATION

(11) **EP 3 357 407 A1**
(43) Date of publication of application: **08.08.2018**
(21) Application number: 16859573.4
(22) Date of filing: 13.10.2016
(51) Int. Cl.: A61B 1/04, G06T 1/00

(54) **ENDOSCOPE IMAGE PROCESSING DEVICE**

(30) Priority: 26.10.2015 JP 2015209891
(71) Applicant: Olympus Corporation, Hachioji-shi Tokyo 192-8507 (JP)
(72) Inventor: IMAIZUMI Katsuichi, Hachioji-shi Tokyo 192-8507 (JP); HASHIMOTO Susumu, Hachioji-shi Tokyo 192-8507 (JP)
(74) Representative: Gunzelmann, Rainer
(86) International application number: PCT/JP2016/080310
(87) International publication number: WO 2017/073338

(57) **Abstract**

An endoscope image processing apparatus includes a detection section 34 configured to receive an observation image G1 of a subject and to detect a feature region L in the observation image G1, based on a predetermined feature value for the observation image G1, a notification section 35a configured to notify, to a surgeon, detection of the feature region L through notification processing and to generate a first display image D1 in a case where the feature region L is detected by the detection section 34, and an enhancement processing section 35b configured to perform enhancement processing on the feature region L and to generate a second display image D2 in the case where the feature region L is detected by the detection section 34.

## Description

### Technical Field

The present invention relates to an endoscope image processing apparatus.

### Background Art

In an endoscope apparatus, a surgeon conventionally watches an observation image and determines presence of a lesion part, etc. To prevent oversight of the lesion part when the surgeon watches the observation image, for example, as disclosed in Japanese Patent Application Laid-Open Publication No. 2011-255006, an endoscope image processing apparatus that adds an alert image to a region of interest detected through image processing and displays the observation image has been proposed.

In the existing endoscope image processing apparatus, however, the alert image may be displayed before the surgeon discovers the lesion part, which may deteriorate attentiveness of the surgeon to a region not indicated by the alert image, and may discourage the surgeon from discovering a lesion part through visual observation to inhibit improvement of lesion part discovering ability.

Accordingly, an object of the present invention is to provide an endoscope image processing apparatus that presents the region of interest to the surgeon while suppressing deterioration of attentiveness of the surgeon to the observation image and not inhibiting improvement of the lesion part discovering ability.

### Disclosure of Invention

### Means for Solving the Problem

An endoscope image processing apparatus according to an aspect of the present invention includes a detection section configured to receive an observation image of a subject and to detect a feature region in the observation image, based on a predetermined feature value for the observation image, a notification section configured to notify, to a surgeon, detection of the feature region through notification processing and to generate a first display image in a case where the feature region is detected by the detection section, and an enhancement processing section configured to perform enhancement processing on the feature region and to generate a second display image in the case where the feature region is detected by the detection section.

### Brief Description of the Drawings

Fig. 1 is a block diagram illustrating a schematic configuration of an endoscope system according to a first embodiment of the present invention;
Fig. 2 is a block diagram illustrating a configuration of a detection support unit of the endoscope system according to the first embodiment of the present invention;
Fig. 3 is an explanatory diagram to explain an example of a screen configuration of a display image of the endoscope system according to the first embodiment of the present invention;
Fig. 4 is explanatory diagram to explain an example of screen transition of the endoscope system according to the first embodiment of the present invention;
Fig. 5 is an explanatory diagram to explain an example of a screen configuration of a second display image of an endoscope system according to a modification 1 of the first embodiment of the present invention;
Fig. 6 is an explanatory diagram to explain an example of screen transition of the endoscope system according to the modification 1 of the first embodiment of the present invention;
Fig. 7 is an explanatory diagram to explain an example of a screen configuration of a first display image of an endoscope system according to a modification 2 of the first embodiment of the present invention;
Fig. 8 is a block diagram illustrating configurations of a detection support unit and an operation section of an endoscope system according to a second embodiment of the present invention;
Fig. 9 is an explanatory diagram to explain an example of a screen configuration of a display image of an endoscope system according to a third embodiment of the present invention;
Fig. 10 is a block diagram illustrating a configuration of a detection support unit of an endoscope system according to a fourth embodiment of the present invention; and
Fig. 11 is an explanatory diagram to explain an example of a screen configuration of a synthesized image of the endoscope system according to the fourth embodiment of the present invention.

### Best Mode for Carrying Out the Invention

Embodiments of the present invention are described below with reference to drawings.

### (First Embodiment)

### (Configuration)

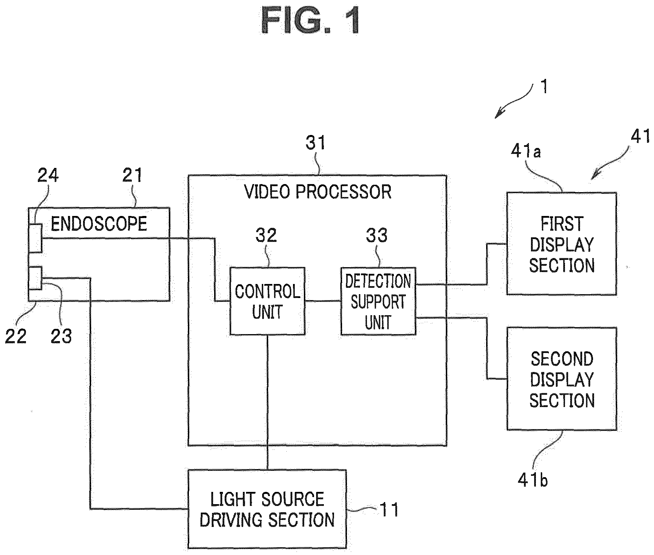

Fig. 1 is a block diagram illustrating a schematic configuration of an endoscope system 1 according to a first embodiment of the present invention.

The endoscope system 1 includes a light source driving section 11, an endoscope 21, a video processor 31, and a display unit 41. The light source driving section 11 is connected to the endoscope 21 and the video processor 31. The endoscope 21 is connected to the video processor 31. The video processor 31 is connected to the display unit 41. The display unit 41 includes a first display section 41a and a second display section 41b as described later.

The light source driving section 11 is a circuit that drives an LED 23 provided at a distal end of an insertion section 22 of the endoscope 21. The light source driving section 11 is connected to a control unit 32 of the video processor 31 and the LED 23 of the endoscope 21. The light source driving section 11 is configured to receive a control signal from the control unit 32, to output a driving signal to the LED 23, and to drive the LED 23 to emit light.

The endoscope 21 is configured such that the insertion section 22 is inserted into a subject and an image inside the subject is picked up. The endoscope 21 includes an image pickup section including the LED 23 and an image pickup device 24.

The LED 23 is provided in the insertion section 22 of the endoscope 21, and is configured to apply illumination light to the subject under control of the light source driving section 11.

The image pickup device 24 is provided in the insertion section 22 of the endoscope 21 and is disposed so as to take in reflected light of the subject that has been irradiated with light, through an unillustrated observation window.

The image pickup device 24 photoelectrically converts the reflected light of the subject taken in through the observation window, and converts an analog image pickup signal into a digital image pickup signal by an unillustrated AD converter, thereby outputting the digital image pickup signal to the video processor 31.

The video processor 31 is an endoscope image processing apparatus including an image processing circuit. The video processor 31 includes the control unit 32 and the detection support unit 33.

The control unit 32 can transmit the control signal to the light source driving section 11 to drive the LED 23.

The control unit 32 can perform, on the image pickup signal provided from the endoscope 21, image adjustment such as gain adjustment, white balance adjustment, gamma correction, contour emphasis correction, and magnification/reduction adjustment, and can sequentially output an observation image G1 of the subject described later, to the detection support unit 33.

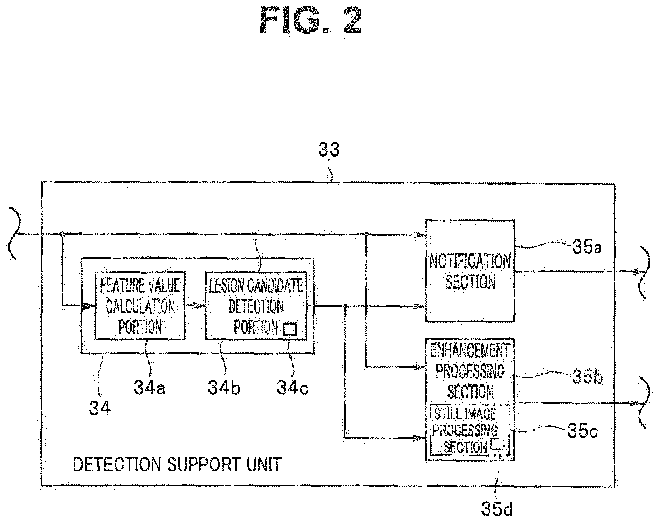

Fig. 2 is a block diagram illustrating a configuration of the detection support unit 33 of the endoscope system 1 according to the first embodiment of the present invention. As illustrated in Fig. 2, the detection support unit 33 includes a detection section 34, a notification section 35a, and an enhancement processing section 35b.

The detection section 34 is a circuit that receives the observation image G1 of the subject and detects a lesion candidate region L that is a feature region in the observation image G1, based on a predetermined feature value for the observation image G1. The detection section 34 includes a feature value calculation portion 34a and a lesion candidate detection portion 34b.

The feature value calculation portion 34a is a circuit that calculates the predetermined feature value for the observation image G1 of the subject. The feature value calculation portion 34a is connected to the control unit 32 and the lesion candidate detection portion 34b. The feature value calculation portion 34a can calculate the predetermined feature value from the observation image G1 of the subject sequentially provided from the control unit 32, and can output the predetermined feature value to the lesion candidate detection portion 34b.

The predetermined feature value is obtained by calculating a variation between each pixel in a predetermined small region and a pixel adjacent to each pixel for each predetermined small region in the observation image G1, namely, an inclination value. Note that the predetermined feature value is not limited to the value calculated from the inclination value with the adjacent pixel, and may be a numerical value of the observation image G1 obtained by another method.

The lesion candidate detection portion 34b is a circuit that detects the lesion candidate region L in the observation image G1 from information of the predetermined feature value. The lesion candidate detection portion 34b includes a ROM 34c to previously hold a plurality of pieces of polyp model information. The lesion candidate detection portion 34b is connected to the notification section 35a and the enhancement processing section 35b.

The polyp model information includes a feature value of feature common to a large number of polyp images.

The lesion candidate detection portion 34b detects the lesion candidate region L, based on the predetermined feature value provided from the feature value calculation portion 34a and the plurality of pieces of polyp model information, and outputs lesion candidate information to the notification section 35a and the enhancement processing section 35b.

More specifically, when the lesion candidate detection portion 34b compares the predetermined feature value for each predetermined small region provided from the feature value calculation portion 34a with the feature value of the polyp model information held by the ROM 34c, and both feature values are coincident with each other, the lesion candidate detection portion 34b detects the lesion candidate region L. When the lesion candidate region L is detected, the lesion candidate detection portion 34b outputs, to the notification section 35a and the enhancement processing section 35b, the lesion candidate information that includes position information and size information of the detected lesion candidate region L.

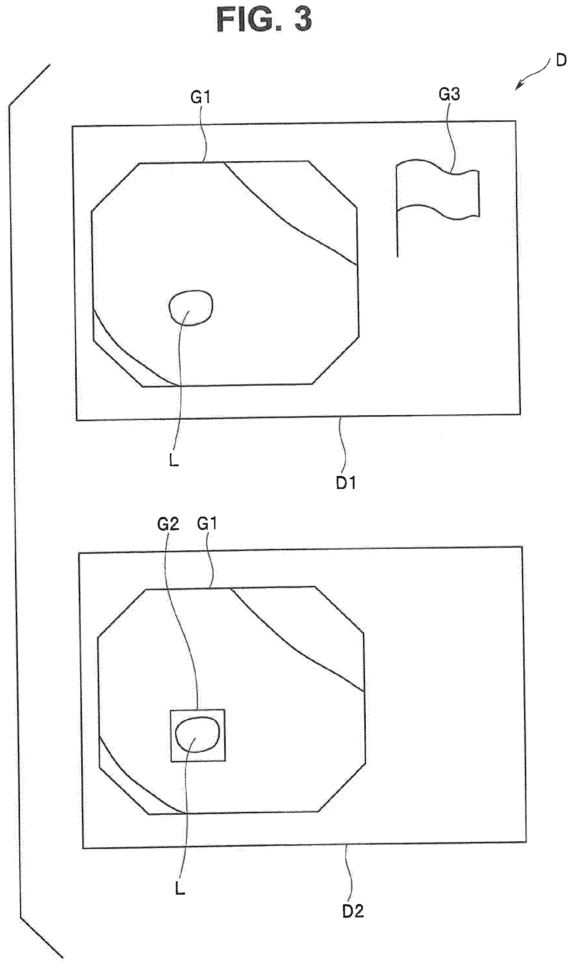

Fig. 3 is an explanatory diagram to explain an example of a screen configuration of a display image D of the endoscope system 1 according to the first embodiment of the present invention.

The display image D of the endoscope system 1 includes a first display image D1 and a second display image D2. The first display image D1 is generated by the notification section 35a. The second display image D2 is generated by the enhancement processing section 35b. The observation image G1 is disposed in each of the first display image D1 and the second display image D2. In Fig. 3, the observation image G1 shows an inner wall of a large intestine including the lesion candidate region L as an example.

The notification section 35a is a circuit that notifies detection of the lesion candidate region L to a surgeon through notification processing and generates the first display image D1 in a case where the lesion candidate region L is detected in the observation image G1 by the detection section 34. The notification section 35a is connected to the first display section 41a. The notification section 35a generates the first display image D1 based on the lesion candidate information provided from the lesion candidate detection portion 34b and the observation image G1 provided from the control unit 32, and outputs the first display image D1 to the first display section 41a.

The notification processing is processing to display a notification image G3 in a region other than the lesion candidate region L. In Fig. 3, the notification image G3 with a flag pattern is illustrated as an example. Alternatively, for example, the notification image G3 may be a triangle, a circle, or a star.

The enhancement processing section 35b is a circuit that performs enhancement processing on the lesion candidate region L and generates the second display image D2 in the case where the lesion candidate region L is detected in the observation image G1 by the detection section 34. The enhancement processing section 35b is connected to the second display section 41b that is separated from the first display section 41a. The enhancement processing section 35b generates the second display image D2 based on the lesion candidate information provided from the lesion candidate detection portion 34b and the observation image G1 provided from the control unit 32, and outputs the second display image D2 to the second display section 41b.

The enhancement processing is processing to perform display indicating the position of the lesion candidate region L. More specifically, the enhancement processing is processing to add a marker image G2 that surrounds the lesion candidate region L, to the observation image G1 provided from the control unit 32, based on the position information and the size information included in the lesion candidate information. Note that, in Fig. 3, the marker image G2 has a square shape as an example. Alternatively, for example, the marker image G2 may be a triangle, a circle, or a star. Further, in Fig. 3, the marker image G2 is a frame image surrounding the lesion candidate region L as an example. The marker image G2, however, may be an image not surrounding the lesion candidate region L as long as the marker image G2 indicates the position of the lesion candidate region L. For example, the position of the lesion candidate region L may be indicated with brightness or a color tone different from brightness or a color tone of peripheral regions.

The display unit 41 is configured to display, on the screen, the display image D provided from the detection support unit 33. The display unit 41 includes the first display section 41a and the second display section 41b that are monitors separated from each other. More specifically, the first display section 41a displays the first display image D1 provided from the notification section 35a, and the second display section 41b displays the second display image D2 provided from the enhancement processing section 35b.

### (Action)

Operation of the endoscope system 1 is described.

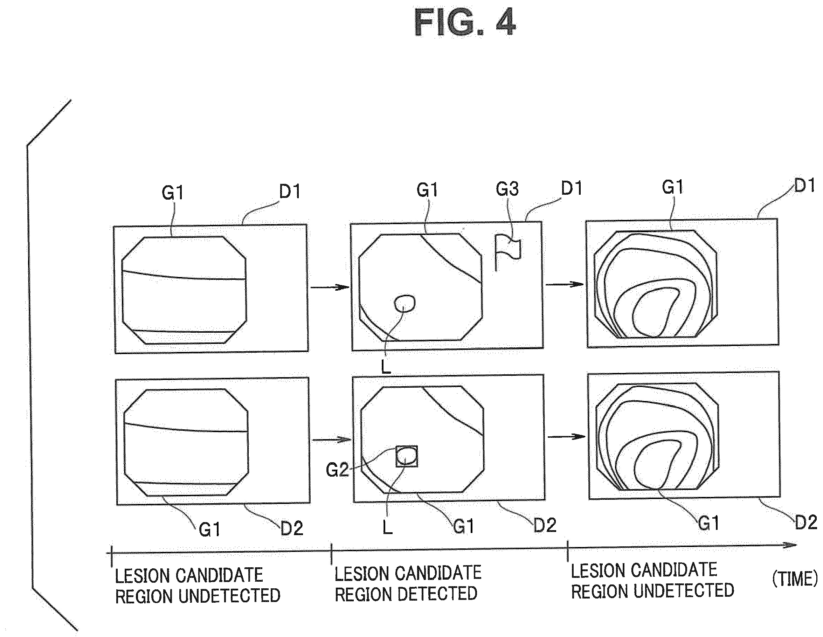

Fig. 4 is an explanatory diagram to explain an example of screen transition in the endoscope system 1 according to the first embodiment of the present invention.

First, the observation of the subject through the endoscope 21 is started. When the lesion candidate region L is not detected by the lesion candidate detection portion 34b, the notification section 35a generates the first display image D1 without performing the notification processing and outputs the first display image D1 to the first display section 41a, and the enhancement processing section 35b generates the second display image D2 without performing the enhancement processing and outputs the second display image D2 to the second display section 41b, both based on the observation image G1 provided from the control unit 32.

Subsequently, when the lesion candidate region L is detected by the lesion candidate detection portion 34b, the notification section 35a performs the notification processing, then generates the first display image D1, and outputs the first display image D1 to the first display section 41a, and the enhancement processing section 35b performs the enhancement processing, then generates the second display image D2, and outputs the second display image D2 to the second display section 41b, both based on the lesion candidate information provided from the lesion candidate detection portion 34b and the observation image G1 provided from the control unit 32.

Subsequently, when the lesion candidate region L is no longer detected, the notification section 35a generates the first display image D1 without performing the notification processing and outputs the first display image D1 to the first display section 41a, and the enhancement processing section 35b generates the second display image D2 without performing the enhancement processing and outputs the second display image D2 to the second display section 41b, both based on the observation image G1 provided from the control unit 32.

As a result, the surgeon can observe the first display image D1 displayed on the first display section 41a serving as a main screen, and can observe the second display image D2 displayed on the second display section 41b serving as a sub-screen as necessary. For example, when the surgeon observes the subject with the first display image D1 and the notification image G3 is displayed on the first display image D1, the surgeon can observe the first display image D1 more carefully and the surgeon himself/herself can discover a lesion part through visual observation. Further, when the surgeon cannot discover a lesion part in the first display image D1, the surgeon can turn eyes on the second display image D2 as necessary, and can confirm the lesion candidate region L more carefully based on the display position of the marker image G2.

According to the above-described first embodiment, the marker image G2 is displayed in the second display image D2, which makes it possible to present a region of interest to the surgeon while suppressing deterioration of attentiveness of the surgeon to the first display image D1 and not inhibiting improvement of the lesion part discovering ability.

### (Modification 1 of First Embodiment)

In the above-described first embodiment, the second display image D2 includes the observation image G1 that is displayed as a movie. Alternatively, the second display image D2 may include the observation image G1 and a still image G4.

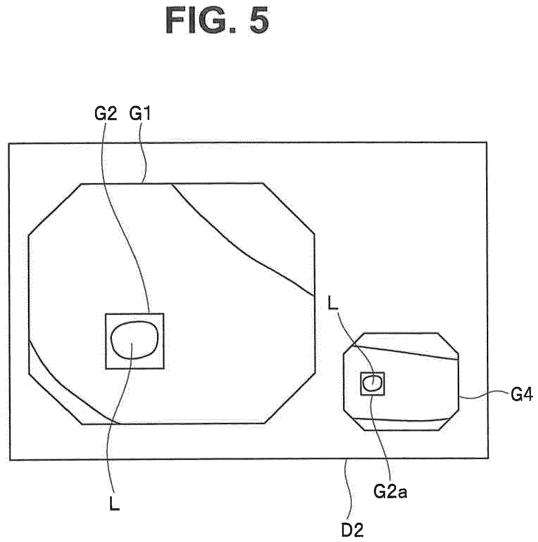

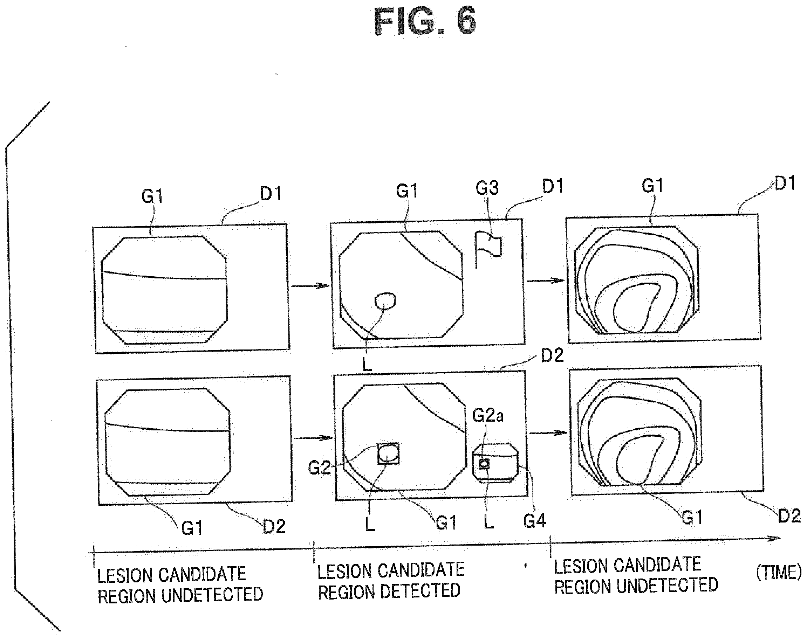

Fig. 5 is an explanatory diagram to explain an example of a screen configuration of the second display image D2 of the endoscope system 1 according to a modification 1 of the first embodiment of the present invention. Fig. 6 is an explanatory diagram to explain an example of screen transition of the endoscope system 1 according to the modification 1 of the first embodiment of the present invention. In description of the modification 1 of the first embodiment, the components same as the components in the first embodiment are denoted by the same reference numerals and description of the components is omitted.

In the modification 1 of the first embodiment, the enhancement processing section 35b includes a still image processing section 35c and a memory 35d (alternate long and short dash line in Fig. 2).

The still image processing section 35c is a circuit that can generate a still image G4 that is obtained by performing the enhancement processing on the lesion candidate region L detected in an observation image provided before the observation image G1.

The memory 35d is configured to temporarily hold the still image G4.

When the lesion candidate region L is detected by the lesion candidate detection portion 34b, the enhancement processing section 35b causes the memory 35d to temporarily hold the still image G4. The enhancement processing section 35b adds a marker image G2a to the still image G4 temporarily held by the memory 35d, and causes the second display section 41b to display the still image G4.

When the lesion candidate region L is no longer detected, the enhancement processing section 35b hides the still image G4.

According to the above-described modification 1 of the first embodiment, it is possible to more surely indicate, to the surgeon, the position of the lesion candidate region L by the still image G4 in the second display image D2. In addition, it is possible to present the region of interest to the surgeon while suppressing deterioration of attentiveness of the surgeon to the first display image D1 and not inhibiting improvement of the lesion part discovering ability.

### (Modification 2 of First Embodiment)

In the first embodiment and the modification 1 of the first embodiment, the notification section 35a displays the notification image G3 in the region other than the lesion candidate region L. Alternatively, an image G5 that surrounds the observation image G1 may be displayed.

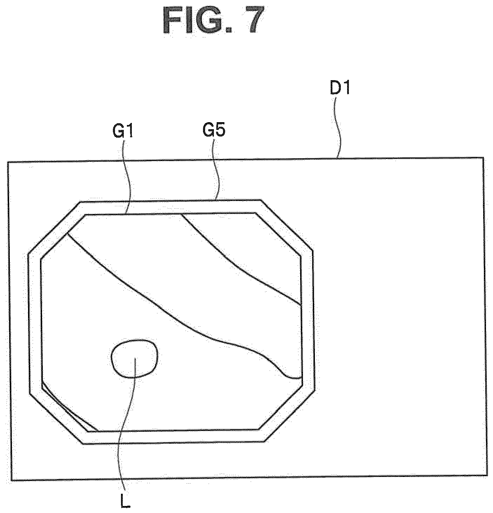

Fig. 7 is an explanatory diagram to explain an example of the screen configuration of the first display image D1 of the endoscope system 1 according to a modification 2 of the first embodiment of the present invention. In description of the modification 2 of the first embodiment, the components same as the components in the first embodiment and the modification 1 of the first embodiment are denoted by the same reference numerals and description of the components is omitted.

In the modification 2 of the first embodiment, the notification section 35a is configured to display the image G5 that surrounds the observation image G1 through the notification processing when the lesion candidate region L is detected by the lesion candidate detection portion 34b.

According to the configuration, the display of the image G5 surrounding the observation image G1 in the first display image D1 allows the surgeon to easily find detection of the lesion candidate region L by the lesion candidate detection portion 34b even when the surgeon pays attention to any part of the first display image D1.

### (Second Embodiment)

In the first embodiment, the modification 1 of the first embodiment, and the modification 2 of the first embodiment, the first display image D1 generated by the notification section 35a is outputted to the first display section 41a, and the second display image D2 generated by the enhancement processing section 35b is outputted to the second display section 41b; however, an output destination of the first display image D1 and an output destination of the second display image D2 may be exchanged with each other.

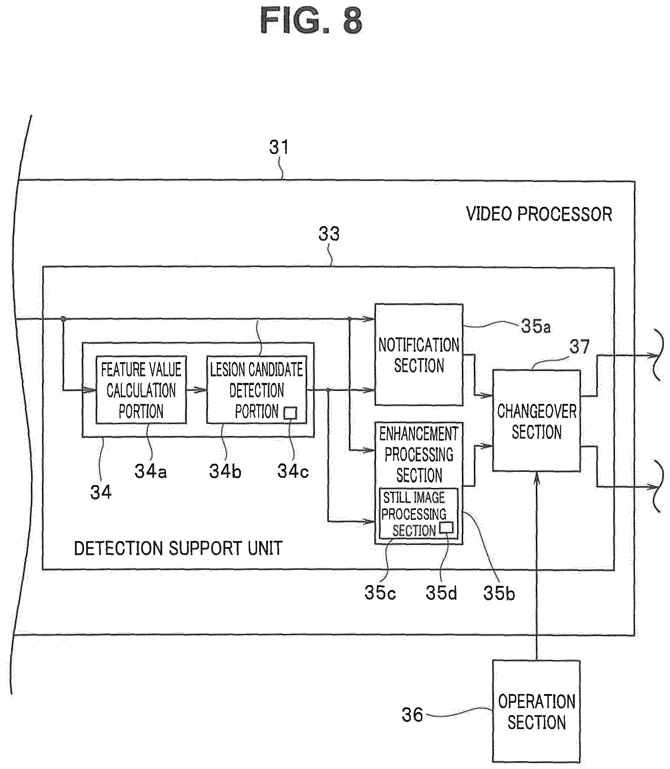

Fig. 8 is a block diagram illustrating configurations of the detection support unit 33 and an operation section 36 of the endoscope system 1 according to a second embodiment of the present invention. In description of the second embodiment, the components same as the components in the first embodiment, the modification 1 of the first embodiment, and the modification 2 of the first embodiment are denoted by the same reference numerals and description of the components is omitted.

In the second embodiment, the endoscope system 1 includes the operation section 36 and a changeover section 37.

The operation section 36 includes a changeover switch that receives an instruction of the surgeon. The operation section 36 is connected to the changeover section 37. When the operation section 36 receives an instruction for changeover of the image output destination from the surgeon, the operation section 36 outputs a changeover instruction signal to the changeover section 37.

The changeover section 37 is a circuit that can exchange the output destination of the first display image D1 and the output destination of the second display image D2 with each other. The changeover section 37 is connected to the notification section 35a, the enhancement processing section 35b, the first display section 41a, and the second display section 41b. When the changeover section 37 receives the changeover instruction signal from the operation section 36, the changeover section 37 exchanges the output destination of the first display image D1 provided from the notification section 35a and the output destination of the second display image D2 provided from the enhancement processing section 35b with each other, to set the output destination of the first display image D1 to the second display section 41b and to set the output destination of the second display image D2 to the first display section 41a.

According to the configuration, it is possible to exchange and display the first display image D1 and the second display image D2 added with the marker image G2 without causing the surgeon to switch attention between the first display section 41a and the second display section 41b, which makes it possible to present the region of interest to the surgeon while suppressing deterioration of attentiveness of the surgeon to the first display image D1 and not inhibiting improvement of the lesion part discovering ability.

### (Third Embodiment)

In the first embodiment, the modification 1 of the first embodiment, the modification 2 of the first embodiment, and the second embodiment, the first display image D1 and the second display image D2 have the same size; however, the first display image D1 and the second display image D2 may have sizes different from each other.

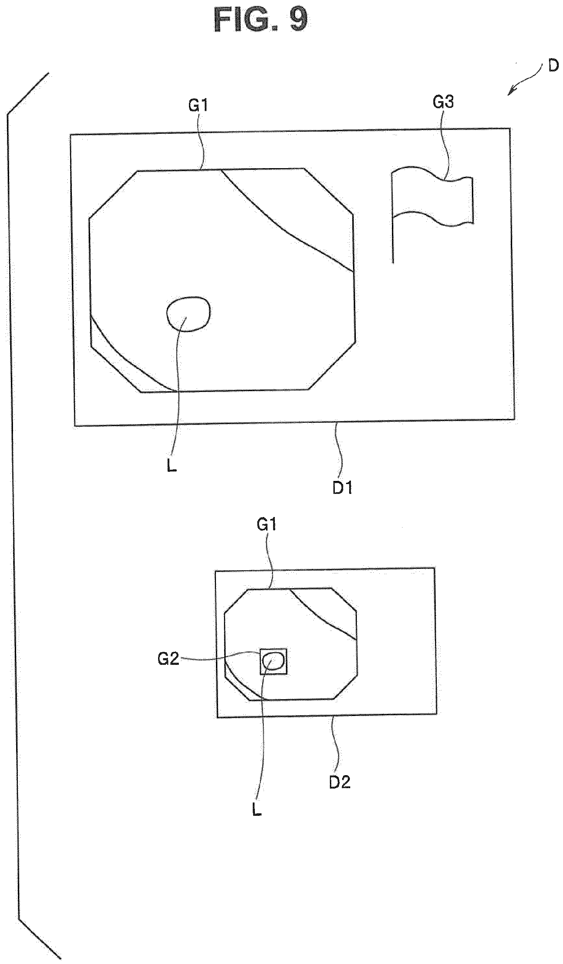

Fig. 9 is an explanatory diagram to explain an example of the screen configuration of the display image D of the endoscope system 1 according to a third embodiment of the present invention. In description of the third embodiment, the components same as the components in the first embodiment, the modification 1 of the first embodiment, the modification 2 of the first embodiment, and the second embodiment are denoted by the same reference numerals and description of the components is omitted.

As illustrated in Fig. 9, in the third embodiment, the first display image D1 has a large size, and the second display image D2 has a small size. In the third embodiment, the display screen of the first display section 41a that displays the first display image D1 may be made larger than the display screen of the second display section 41b that displays the second display image D2. Alternatively, the first display image D1 is generated as an image larger in size than the second display image D2, and the first display image D1 may be displayed larger than the second display image D2 on the first display section 41a and the second display section 41b that include the display screen of the same size.

According to the configuration, the first display image D1 is displayed large and the second display image D2 added with the marker image G2 is displayed small. As a result, the surgeon tends to turn the eyes on the first display image D1. Therefore, it is possible to present the region of interest to the surgeon while suppressing deterioration of attentiveness of the surgeon to the first display image D1 and not inhibiting improvement of the lesion part discovering ability.

### (Fourth Embodiment)

In the first embodiment, the modification 1 of the first embodiment, the modification 2 of the first embodiment, the second embodiment, and the third embodiment, the first display image D1 and the second display image D2 are respectively displayed on the first display section 41a and the second display section 41b that are separated from each other. Alternatively, the first display image D1 and the second display image D2 may be displayed by one display section.

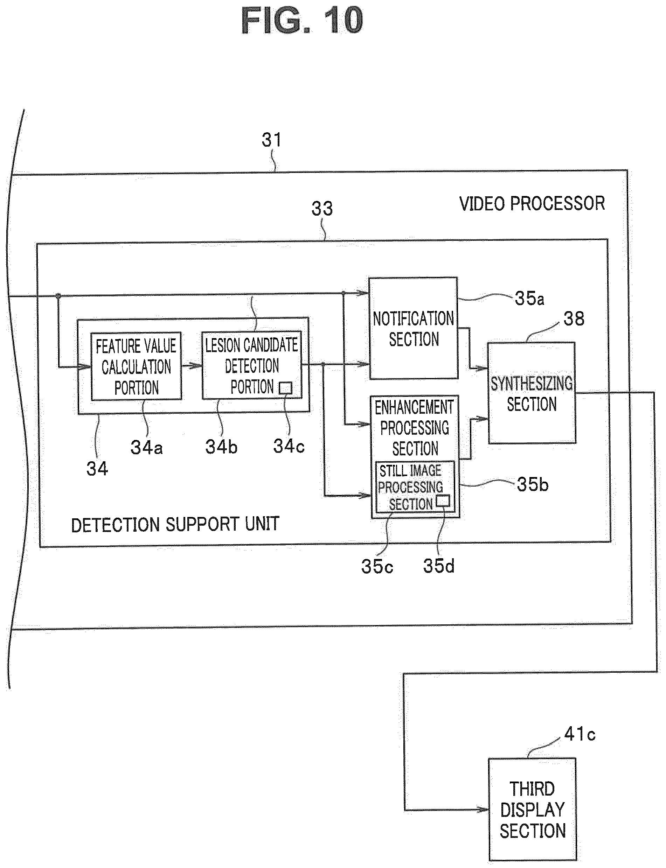

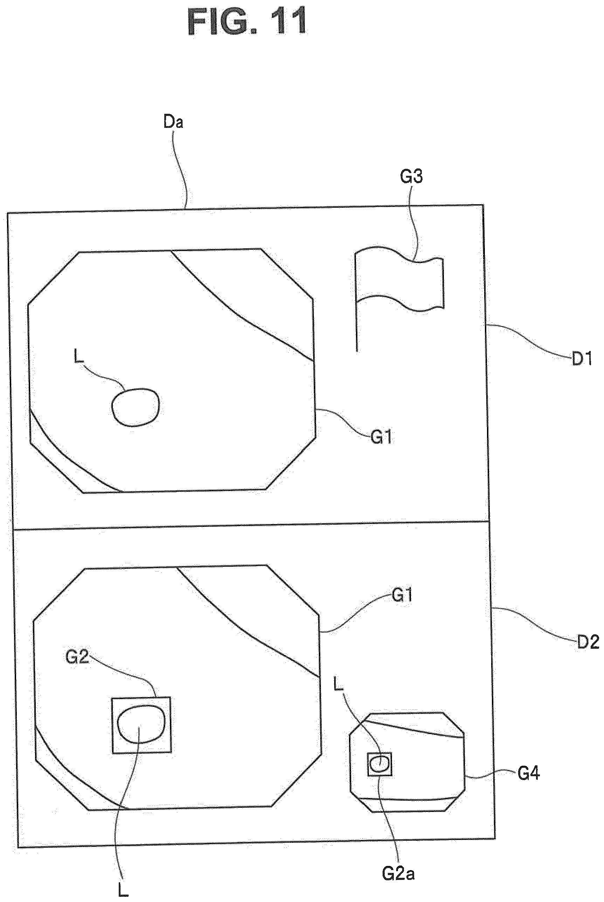

Fig. 10 is a block diagram illustrating a configuration of the detection support unit 33 of the endoscope system 1 according to a fourth embodiment of the present invention. Fig. 11 is an explanatory diagram to explain an example of a screen configuration of a synthesized image Da of the endoscope system 1 according to the fourth embodiment of the present invention. In description of the fourth embodiment, the components same as the components in the first embodiment, the modification 1 of the first embodiment, the modification 2 of the first embodiment, the second embodiment, and the third embodiment are denoted by the same reference numerals and description of the components is omitted.

In the fourth embodiment, the detection support unit 33 includes a synthesizing section 38.

The synthesizing section 38 is a circuit that can synthesize the first display image D1 and the second display image D2 to generate the synthesized image Da. The synthesizing section 38 is connected to the notification section 35a, the enhancement processing section 35b, and a third display section 41c. The synthesizing section 38 synthesizes the first display image D1 provided from the notification section 35a and the second display image D2 provided from the enhancement processing section 35b to generate the synthesized image Da illustrated in Fig. 11 as an example, thereby outputting the synthesized image Da to the third display section 41c.

According to the configuration, it is possible to display, on the third display section 41c, the synthesized image Da that is obtained by synthesizing the first display image D1 and the second display image D2 added with the marker image G2, and to present the region of interest to the surgeon while suppressing deterioration of attentiveness of the surgeon to the first display image D1 and not inhibiting improvement of the lesion part discovering ability.

According to the above-described embodiments, it is possible to present the region of interest to the surgeon while suppressing deterioration of attentiveness of the surgeon to the observation image G1 and not inhibiting improvement of the lesion part discovering ability.

Note that, in the embodiments, one lesion candidate region L is displayed in the observation image G1 for description; however, a plurality of lesion candidate regions L may be displayed in the observation image G1 in some cases. In this case, the notification processing and the enhancement processing are performed on each of the lesion candidate regions L.

Note that, in the embodiments, the notification section 35a displays the notification image G3 to perform notification to the surgeon; however, the notification section 35a may generate sound from an unillustrated speaker to perform notification to the surgeon.

Note that, in the embodiments, the control unit 32 performs, on the image pickup signal provided from the endoscope 21, the image adjustment such as gain adjustment, white balance adjustment, gamma correction, contour emphasis correction, and magnification/reduction adjustment, and provides the image-adjusted observation image G1 to the detection support unit 33. A part or all of the image adjustment, however, may be performed not on the image pickup signal before being provided to the detection support unit 33 but on the image signal outputted from the detection support unit 33.

Note that, in the embodiments, the enhancement processing section 35b adds the marker image G2 to the lesion candidate region L; however, the marker image G2 may be color-coded and displayed depending on a degree of certainty of the detected lesion candidate region L. In this case, the lesion candidate detection portion 34b outputs, to the enhancement processing section 35b, the lesion candidate information including degree of certainty information of the lesion candidate region L, and the enhancement processing section 35b performs the enhancement processing using the color coding based on the degree of certainty information of the lesion candidate region L. The configuration allows the surgeon to estimate degree of possibility of false positive (false detection) based on the color of the marker image G2 when the surgeon observes the lesion candidate region L.

Note that, in the embodiments, the detection support unit 33 is disposed inside the video processor 31; however, the detection support unit 33 may be disposed outside the video processor 31, for example, between the video processor 31 and the display unit 41.

Note that, in the embodiments, the detection support unit 33 includes the circuit; however, each function of the detection support unit 33 may include a processing program, the function of which is achieved through processing by the CPU.

The present invention is not limited to the above-described embodiments, and may be variously modified or alternated without departing from the scope of the present invention.

The present invention makes it possible to provide the endoscope image processing apparatus that presents the region of interest to the surgeon while suppressing deterioration of attentiveness of the surgeon to the observation image and not inhibiting improvement of the lesion part discovering ability.

The present application is based upon and claims the benefit of priority of Japanese Patent Application No. 2015-209891 filed in Japan on October 26, 2015, the disclosed contents of which are incorporated in the specification and the claims of the present application by reference.

## Claims

1. An endoscope image processing apparatus, comprising:
a detection section configured to receive an observation image of a subject and to detect a feature region in the observation image, based on a predetermined feature value for the observation image;
a notification section configured to notify, to a surgeon, detection of the feature region through notification processing and to generate a first display image in a case where the feature region is detected by the detection section; and
an enhancement processing section configured to perform enhancement processing on the feature region and to generate a second display image in the case where the feature region is detected by the detection section.

2. The endoscope image processing apparatus according to claim 1, wherein the notification processing is processing to display a notification image in a region other than the feature region.

3. The endoscope image processing apparatus according to claim 1, wherein the enhancement processing is processing to perform display indicating a position of the feature region.

4. The endoscope image processing apparatus according to claim 1, further comprising a still image processing section configured to generate a still image that is obtained by performing the enhancement processing on the feature region detected in the observation image inputted before the observation image.

5. The endoscope image processing apparatus according to claim 1, wherein the notification processing is processing to display an image that surrounds the observation image.

6. The endoscope image processing apparatus according to claim 1, wherein the first display image is outputted to a first display section, and
the second display image is outputted to a second display section.

7. The endoscope image processing apparatus according to claim 1, further comprising a changeover section configured to be capable of exchanging an output destination of the first display image and an output destination of the second display image with each other.

8. The endoscope image processing apparatus according to claim 1, wherein the first display image is generated as an image larger in size than the second display image.

9. The endoscope image processing apparatus according to claim 1, further comprising a synthesizing section configured to be capable of synthesizing the first display image and the second display image to generate a synthesized image, wherein
the synthesized image synthesized by the synthesizing section is outputted to a third display section.

10. The endoscope image processing apparatus according to claim 1, wherein the detection section is configured to output degree of certainty information of the detected feature region, and
the enhancement processing section performs the enhancement processing using color coding based on the degree of certainty information.
